(19) Europäisches Patentamt
European Patent Office
Office européen des brevets

(11) **EP 3 001 193 A1**

(12) **EUROPEAN PATENT APPLICATION**

(43) Date of publication:
**30.03.2016 Bulletin 2016/13**

(51) Int Cl.:
***G01N 33/18*** (2006.01)     ***G01N 27/48*** (2006.01)

(21) Application number: **15186999.7**

(22) Date of filing: **25.09.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **26.09.2014 IT TO20140765**

(71) Applicant: **Puretech S.r.l.**
**00047 Marino (IT)**

(72) Inventors:
• **SANTONICO, Marco**
**00047 MARINO (IT)**
• **PENNAZZA, Giorgio**
**00047 MARINO (IT)**
• **AMADEI, Federica**
**00047 MARINO (IT)**
• **PUNZO, Gianpaolo**
**00047 MARINO (IT)**

(74) Representative: **Boggio, Luigi et al**
**Studio Torta S.p.A.**
**Via Viotti, 9**
**10121 Torino (IT)**

(54) **SYSTEM FOR CHARACTERISATION OF PURE AND ULTRAPURE WATER**

(57)     The present invention concerns a system (1) for the characterization of pure and ultrapure water. Said system (1) comprises: a signal generator (2) configured to generate an electrical voltage having a predetermined waveform such that to allow the performance of measurements based on cyclic voltammetry; a measuring device (3;8;8') configured to perform measurements based on cyclic voltammetry on a water sample under examination (6) on the basis of the electrical voltage generated by the signal generator (2); and processing means (4) configured to characterize the water sample under examination (6) on the basis of measurements based on cyclic voltammetry performed by the measuring device (3;8;8') on said water sample under examination (6).

FIG. 3

Description

## TECHNICAL FIELD OF THE INVENTION

The present invention concerns, in general, a system for pure and ultrapure water characterization and, in particular, a system designed to use a cyclic voltammetry technique to determine whether a water sample under examination is or not a pure or ultrapure water sample and, preferably, also to provide a microbiological characterization of said water sample under examination.

Conveniently, the present invention can be advantageously exploited for the microbiological characterization of pure and ultrapure water intended, for example, for haemodialysis.

## STATE OF THE ART

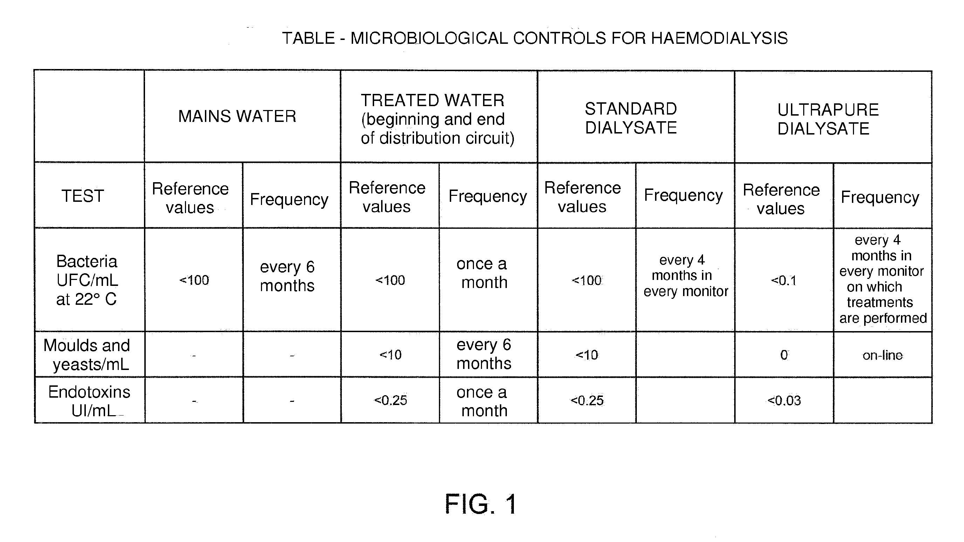

As is known, the water used to supply a haemodialysis centre requires particular treatment procedures and a complete system for production and distribution of the water in order to achieve the best technical, economic and therapeutic results. A patient in dialysis comes into contact weekly with a considerable quantity of water via the dialysis bath, on average 350 litres. It is therefore essential for this solution to have a high quality and purity in terms of correct electrolytic composition, low concentration or absence of organic and inorganic chemical pollutants, low concentration or absence of bacteria, yeasts, fungi and endotoxins. The microbiological purity depends on numerous factors, such as the characteristics of the mains water, the composition and architecture of the water production and distribution plant, the procedures for and frequency of the plant and dialysis monitor disinfection cycles. In said regard, Figure 1 shows a table summarizing the parameters to be controlled, the relative control frequencies and the sampling sites.

As is known, in Italy the Legislative Decree no. 31 of 2 February 2001 (which came into force on 25 December 2003) regulates the quality of water for human consumption in order to protect human health from the negative effects deriving from contamination of water for human consumption, thereby guaranteeing the healthiness and cleanness thereof. In particular, said Legislative Decree establishes the parameters (and relative reference values) that must be taken into account to define the chemical and microbiological quality of water for human consumption.

Furthermore, the chemical and microbiological quality of water intended for medical and biomedical treatments, for example haemodialysis, is generally defined on the basis of a plurality of international reference standards, for example:

- the D1193 and D5196 standards of the ASTM international organization (where ASTM stands for American Society for Testing and Materials);

- the CAP/NCCLS 1988 standard (where CAP stands for College of American Pathologists and NCCLS stands for National Committee for Clinical Laboratory Standards);
- the "International Pharmacopoeia" and "European Pharmacopoeia" standards; and
- the standard for the definition of pure and ultrapure water of the Association for the Advancement of Medical Instrumentation (AAMI).

Therefore, when the following part of the description talks about pure or ultrapure water, reference will be made to water which has chemical and microbiological properties, i.e. characteristics, such as to meet the requirements defined in at least one of the above-mentioned standards in relation to the chemical and microbiological characterization of the pure and ultrapure water.

Currently the search for bacterial microorganisms is carried out using particular analytical techniques. The standard techniques are generally based on the possibility of cultivating the bacteria on suitable substrates and in suitable cultural conditions. The plate counting technique entails the seeding, on culture media, of samples of the water to be examined. The bacterial colonies that develop on the culture medium allow identification of the number of microorganisms present in a known volume of the sample. The waiting times are fairly long, but necessary for the complete growth of the bacterial colonies. In particular, said examinations can last from 18 to 48 hours.

The article by Setterington E. B. et al. entitled "Rapid electrochemical detection of polyaniline-labeled Escherichia coli O157:H7", Biosensors and Bioelectronics, Elsevier BV, NL, vol. 26, No. 5,, 15 January 2011, pages 2208-2214, presents a method and a system for identification of the bacterium Escherichia coli O157:H7. In particular, according to said article by Setterington et al., cells of Escherichia coli O157:H7 are isolated by means of immunomagnetic separation - IMS, labelled with biofunctionalized electroactive polyaniline (immuno-PANI) and detected via cyclic voltammetry implemented by means of screen-printed carbon electrodes.

Also the article by Agnes Obuchowska entitled "Quantitation of bacteria through absorption of intracellular biomolecules on carbon paste and screen-printed carbon electrodes and voltammetry of redox-active probes", Analytical and Bioanalytical Chemistry, Springer, Berlin, DE, vol. 390, No. 5, 20 January 2008, pages 1361-1371, presents an electrochemical method for determining the quantity of bacteria, in particular Escherichia coli JM105, Micrococcus luteus and Clostridium sporogenes, via cyclic voltammetry and by means of screen-printed carbon electrodes.

Furthermore, the American patent application US 2006/0228738 A1 describes a DNA-polypyrrole based biosensor and methods for using said biosensor for rapid detection of the bacterium Escherichia coli and other microorganisms. In particular, the biosensor according to US 2006/0228738 A1 comprises an electrochemical cell which is used to implement a cyclic voltammetry technique and which includes a platinum working electrode, an Ag/AgCl reference electrode and a carbon rod counter electrode.

Lastly, chapter 10 "Bioelectronics for Amperometric Biosensors" by Jaime Punter Villagrasa et al. of the book "State of the Art in Biosensors - General Aspects", 13 March 2013, InTech, provides an overview of the state of the art in the field of biosensors and bioelectronics.

## OBJECT AND SUMMARY OF THE INVENTION

A first object of the present invention is, therefore, to provide a system for characterization of pure and ultrapure water which, in general, overcomes, at least in part, the technical problems of the methods currently used for the characterization of pure and ultrapure water and which, in particular, is able to perform characterization of pure and ultrapure water with a greater rapidity than the methods currently used for the characterization of pure and ultrapure water.

Furthermore, a second object of the present invention is to provide a system for performing characterization of pure and ultrapure water at low cost and in an efficient, reliable and reproducible manner.

These and other objects are achieved by the present invention which concerns a system for pure and ultrapure water characterization, as defined in the attached claims.

In particular, the system for pure and ultrapure water characterization according to the present invention comprises:

- a signal generator configured to generate an electrical voltage having a predetermined waveform such that to allow to perform measurements based on cyclic voltammetry;
- a measuring device configured to perform measurements based on cyclic voltammetry on a water sample under examination on the basis of the electrical voltage generated by the signal generator; and
- processing means configured to characterize the water sample under examination on the basis of the measurements based on cyclic voltammetry performed by the measuring device on said water sample under examination;

wherein the measuring device comprises:

- an electrochemical cell, which includes

  - a container designed to contain the water sample under examination, and
  - a working electrode, a counter electrode and a reference electrode arranged so as to be immersed, at least partially, in the water sample under examination contained in the container;

- a first operational amplifier, which includes

  - a non-inverting input designed to receive the electrical voltage generated by the signal generator,
  - an inverting input connected to the reference electrode,
  - an output connected to the counter electrode, and
  - a feedback capacity arranged between the inverting input and the output,
    thereby resulting in said first operational amplifier being configured to apply to the electrochemical cell the electrical voltage generated by the signal generator to perform the measurements based on cyclic voltammetry on the water sample under examination; and

- a current-voltage converter which is

  - connected to the working electrode to receive an electrical current induced on said working electrode by the application to the electrochemical cell of the electrical voltage generated by the signal generator, and
  - configured to convert said electrical current into a corresponding electrical voltage thereby providing an electrical output voltage;
    and wherein the processing means are configured to detect whether the water sample under examination is a pure or ultrapure water sample on the basis of:

- the electrical voltage generated by the signal generator;
- the electrical output voltage supplied by the current-voltage converter; and
- a reference measurement model generated on the basis of measurements based on cyclic voltammetry performed by the measuring device on

  - first reference water samples having first chemical and microbiological characteristics which, according to a given reference standard, characterize pure water, and
  - second reference water samples having second chemical and microbiological characteristics which, according to said given reference standard, characterize ultrapure water.

## BRIEF DESCRIPTION OF THE DRAWINGS

For a better understanding of the present invention, some preferred embodiments, provided purely by way of non-limiting example, will now be illustrated with reference to the accompanying drawings (not all to scale), in which:

- Figure 1 shows a table which summarizes the microbiological controls (in terms of types of controls performed, frequency of the controls and relative reference values) generally performed on the water and on the dialysis bath (or dialysate) intended for haemodialysis;
- Figure 2 schematically illustrates a functional architecture of a system for pure and ultrapure water characterization according to a preferred embodiment of the present invention;
- Figure 3 illustrates in greater detail a component of the system of Figure 2;
- Figure 4 shows a specific preferred embodiment of the system of Figure 2; and
- Figure 5 shows an alternative embodiment of a component of the system of Figure 4.

## DETAILED DESCRIPTION OF PREFERRED EMBODIMENTS OF THE INVENTION

The following description is provided to allow a person skilled in the art to produce and use the invention. Various modifications to the embodiments presented will be immediately evident to persons skilled in the art and the general principles disclosed here could be applied to other embodiments and applications without departing from the scope of protection of the present invention as defined in the attached claims.

Therefore the present invention must not be understood as limited solely to the embodiments described and shown, but must be accorded the widest scope of protection consistent with the principles and characteristics presented here and defined in the attached claims.

The present invention concerns a system designed to use a cyclic voltammetry technique to determine whether a water sample under examination is or not a pure or ultrapure water sample and, preferably, also to provide a microbiological characterization of said water example under examination.

As is known, cyclic voltammetry is a potentiodynamic electrochemical measurement method which involves:

- application, to a solution under examination, of an electrical potential which varies linearly over time and has a cyclic or periodic time trend, in particular via the use of an electrochemical cell comprising three electrodes immersed in the solution under examination, specifically a working electrode (WE), a counter electrode (CE) and a reference electrode (RE); and
- measurement of the current generated in the solution under examination.

Cyclic voltammetry allows both qualitative and quantitative analysis of a solution under examination to be performed (for example it is used to study the properties of an analyte in solution) and is generally based on oxidation-reduction phenomena of electroactive species.

The present invention stems from the innovative idea of the Applicant of exploiting cyclic voltammetry to perform qualitative and quantitative analyses of in vivo species, such as bacteria and/or endotoxins present in a water sample under examination (for example, a water sample intended for haemodialysis) in order to determine the species and relative concentration and, therefore, in order to perform a characterization, in particular a microbiological characterization, of the water sample under examination.

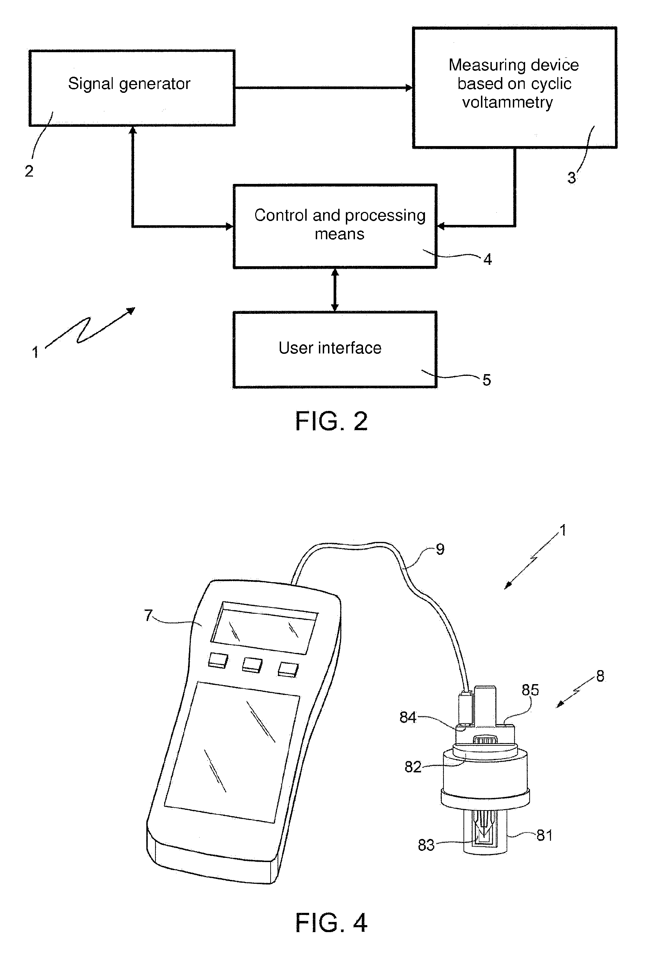

For a better understanding of the present invention, Figure 2 shows a functional block diagram of a system for the characterization of pure and ultrapure water (indicated overall by 1) according to a preferred embodiment of the present invention.

In particular, as shown in Figure 2, the system 1 comprises:

- a signal generator 2 configured to generate an electrical signal having a predetermined waveform such that to allow to perform measurements based on cyclic voltammetry on a water sample under examination (not shown in Figure 2), conveniently an electrical voltage having a predetermined waveform such that to cause, when said electrical voltage is applied to an electrochemical cell (not shown in Figure 2) containing said water sample under examination, oxidation-reduction phenomena in a cyclic manner; said predetermined waveform being preferably a triangular wave;
- a measuring device based on cyclic voltammetry 3, which is connected to the signal generator 2 to receive the electrical signal generated by the latter, and which is configured to perform on the water sample under examination cyclo-voltammetric measurements based on the electrical signal received;
- control and processing means 4, which are

  - coupled to the signal generator 2 and configured to acquire the electrical signal generated by the latter and to control the operation of said signal generator 2, for example to set or modify the above-mentioned predetermined waveform,
  - connected to the measuring device based on cyclic voltammetry 3 and configured to acquire from the latter an electrical signal resulting from the cyclo-voltammetric measurements performed, conveniently an electrical current or voltage, and
  - configured to determine, on the basis of the electrical signals acquired from the signal generator 2 and from the measuring device based on cyclic voltammetry 3 and a predetermined reference measurement model, whether the water sample under examination is a pure or ultrapure water sample and, preferably, also to provide, again on the basis of the electrical signals acquired from the signal generator 2 and from the measuring device based on cyclic voltammetry 3 and the predetermined reference measurement model, a microbiological characterization of the water sample under examination, i.e. to determine microbiological characteristics of said water sample under examination (in terms of bacterial species and/or endotoxins present in said water sample under examination and relative concentrations); and

- a user interface (UI) 5, which is coupled with the control and processing means 4 to exchange commands and data and/or signals with the latter, and which is configured

  - to indicate, for example on a screen/display (not shown in Figure 2), whether the water sample under examination is a pure or ultrapure water sample,
  - preferably also to display, for example again on said screen/display, data indicative of the microbiological characteristics of the water sample under examination, and,
  - even more preferably, also to allow a user to control the operation of the system 1, for example allow a user to set or modify the waveform of the electrical signal generated by the signal generator 2 based on which the cyclo-voltammetric measurements are performed by said measuring device based on cyclic voltammetry 3.

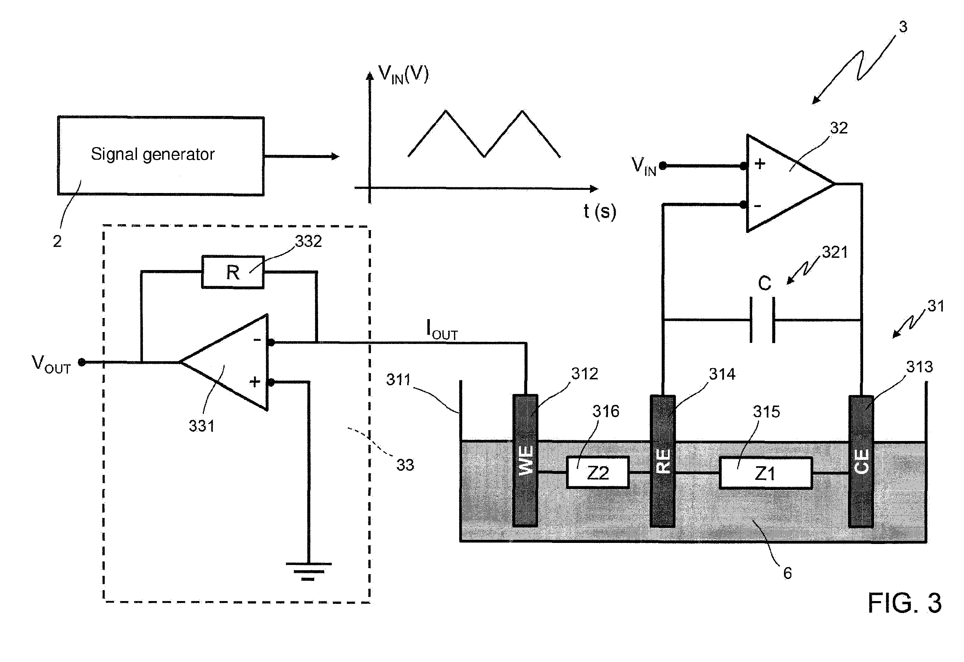

For a better understanding of the operation of the system 1, Figure 3 shows a circuit diagram which represents the measuring device based on cyclic voltammetry 3.

In particular, as shown in Figure 3, said measuring device based on cyclic voltammetry 3 includes:

- an electrochemical cell (indicated as a whole by 31) which comprises

  - an aseptic, i.e. sterile, container (or receptacle) 311 which, in use, contains a water sample under examination 6, and
  - a working electrode WE 312, a counter electrode CE 313 and a reference electrode RE 314 immersed, at least partially, in the water sample under examination 6;

- an operational amplifier 32 comprising a non-inverting input (indicated, in a known manner for a person skilled in the art, by the symbol "+"), an inverting input (indicated, in a known manner for a person skilled in the art, by the sign "-"), an output, and a feedback capacity C (indicated by 321) arranged between the inverting input and the output, wherein

- the non-inverting input, in use, receives the electrical signal generated by the signal generator 2, conveniently, as shown in Figure 3, an electrical voltage $V_{IN}$ having a predetermined triangular waveform (for example with amplitude varying between +1 V and -1 V and frequency of 10 mHz),
- the inverting input is connected to the reference electrode 314, and
- the output is connected to the counter electrode 313; and

- a current-voltage converter 33, which is

  - connected to the working electrode 312 to receive an electrical current $I_{OUT}$ induced on said working electrode 312 by application to the electrochemical cell 31 of the electrical signal provided by the signal generator 2, conveniently by the application to the electrochemical cell 31 of the above-mentioned electrical voltage $V_{IN}$, and
  - configured to convert said electrical current $I_{OUT}$ into a corresponding electrical output voltage $V_{OUT}$ which, in use, is then acquired by the control and processing means 4.

Preferably, the working electrode 312, the counter electrode 313 and the reference electrode 314 are made of gold, platinum and silver chloride respectively.

Conveniently, said current-voltage converter 33 is a transimpedance amplifier (TIA), which includes:

- an operational amplifier 331 which comprises

  - a non-inverting input (indicated, in a manner known to a person skilled in the art, by the symbol "+") connected to ground,
  - an inverting input (indicated, in a manner known to a person skilled in the art, by the sign "-") connected to the working electrode 312 to receive the above-mentioned electrical current $I_{OUT}$, and
  - an output via which, in use, the above-mentioned electrical voltage $V_{OUT}$ is supplied; and

- a feedback resistor R (indicated by 332) arranged between the inverting input and the output of the operational amplifier 331.

Furthermore, Figure 3 also shows two impedances Z1 and Z2 (indicated by 315 and 316 respectively) which represent the impedances present, respectively, between the counter electrode 313 and the reference electrode 314, and between said reference electrode 314 and the working electrode 312.

Therefore, in use, the electrical voltage $V_{IN}$ supplied by the signal generator 2 is applied to the electrochemical cell 31 by means of the operational amplifier 32 which, due to its characteristics of high input impedance and low output impedance, allows said electrical voltage $V_{IN}$ to be taken back the reference electrode 314 thus permitting application of said electrical voltage $V_{IN}$ to the water sample under examination 6. Conveniently, the electrical current $I_{OUT}$ induced on the working electrode 312 by application of the electrical voltage $V_{IN}$ to the electrochemical cell 31 is a current in the order of $\mu$A. As previously described, said current $I_{OUT}$ supplied at the output of the electrochemical cell 31 is converted into the corresponding output voltage $V_{OUT}$ by the transimpedance amplifier 33 in order to improve the stability of the signal and therefore allow correct measurement thereof. In any case, by dividing the output voltage $V_{OUT}$ by the impedance of the measuring circuit, it is possible to obtain the values of the current $I_{OUT}$.

Conveniently, the above-mentioned feedback capacity C 321 can, for example, have a value of 47 pF, the above-mentioned feedback resistor R 332 can, for example, have a value of 15 k$\Omega$, and the operational amplifiers 32 and 331 can be powered (or polarized) with power supply (or polarization) voltages $V^+$ and $V^-$ having, for example, values of +4.5 V and -4.5 V respectively.

Furthermore, in order to provide a measuring device based on cyclic voltammetry 3 which is disposable, inexpensive and compact, the working electrode 312, the counter electrode 313 and the reference electrode 314 are preferably made by means of screen printed electrodes - SPEs.

Preferably, the signal generator 2 is configured to allow setting of the frequency and amplitude values of the triangular wave. For example, the signal generator 2 can conveniently comprise two or more potentiometers that can be activated to set the desired frequency and amplitude values of the triangular wave. In particular, the user interface 5 can be conveniently configured to allow a user to set/select (for example by means of buttons or a touchscreen) the frequency and the amplitude of the triangular waveform of the electrical voltage $V_{IN}$ generated by the signal generator 2 and to supply to the control and processing means 4 corresponding commands indicative of the frequency and amplitude set/selected by the user; furthermore, said control and processing means 4 can be conveniently configured to set, on the signal generator 2, the frequency and amplitude of the triangular waveform of the electrical voltage $V_{IN}$ on the basis of the commands received from the user interface 5, for example by appropriately regulating the above-mentioned potentiometers of the signal generator 2.

Conveniently, the control and processing means 4 are configured to process the electrical signals acquired from the signal generator 2 (i.e. the electrical voltage $V_{IN}$ with triangular waveform) and from the measuring device based on cyclic voltammetry 3 (i.e. the output voltage $V_{OUT}$) so as to obtain a correct acquisition of said electrical signals.

In particular, the control and processing means 4 can conveniently comprise:

- a microcontroller for correct acquisition and storage (and, if necessary, also an appropriate amplitude translation) of the electrical signals acquired;
- low-pass filtering means for filtering the electrical signals acquired;
- analog-digital conversion means for converting the analog electrical signals acquired into corresponding digital signals; and
- a processor configured

  - to determine, on the basis of a predetermined reference measurement model and the electrical signals acquired from the microcontroller, filtered by the low-pass filtering means and converted into digital format by the analog-digital conversion means, whether the water sample under examination 6 is a pure or ultrapure water sample, and,
  - preferably, also to determine, again on the basis of a predetermined reference measurement model and the electrical signals acquired from the microcontroller, filtered by the low-pass filtering means and converted into digital format by the analog-digital conversion means, microbiological characteristics of the water sample under examination 6.

Preferably, the control and processing means 4 are configured to store the above-mentioned predetermined reference measurement model, which is produced during a preliminary phase of training or calibration of the system 1. In particular, during said training/calibration phase, samples of reference water are introduced into the electrochemical cell 31 (specifically into the container 311) of the measuring device based on cyclic voltammetry 3, including samples of pure water and samples of ultrapure water containing known concentrations of bacteria and/or endotoxins (for example, for said purpose it is expedient to use samples of pure and ultrapure water for which a microbiological characterization has been previously obtained by means of one or more known chemical and/or microbiological characterization techniques). In this way, during said training/calibration phase, the control and processing means 4 generate, on the basis of the electrical signals acquired from the signal generator 2 (i.e. the electrical voltage $V_{IN}$ with triangular waveform) and from the measuring device based on cyclic voltammetry 3 (i.e. the output voltage $V_{OUT}$), a reference measurement model which includes first reference measurements associated with samples of pure water containing known concentrations of bacteria and/or endotoxins and second reference measurements associated with samples of ultrapure water containing known concentrations of bacteria and/or endotoxins. Therefore, once the training/calibration phase has been completed, said control and processing means 4, thanks to the reference measurement model produced during said training/calibration phase, are able:

- to determine whether a water sample currently under examination is a sample of pure or ultrapure water, and,
- preferably, to provide a microbiological characterization of said water sample currently under examination.

In particular, the control and processing means 4 are configured to:

- detect that a water sample currently under examination is a sample of pure water if the electrical signals acquired from the signal generator 2 (i.e. the electrical voltage $V_{IN}$ with triangular waveform) and from the measuring device based on cyclic voltammetry 3 (i.e. the output voltage $V_{OUT}$) correspond to one of the first reference measurements of the reference measurement model;
- detect, on the other hand, that a water sample currently under examination is not a pure water sample if the electrical signals acquired from the signal generator 2 and from the measuring device based on cyclic voltammetry 3 do not correspond to one of the first reference measurements of the reference measurement model;
- detect that a water sample currently under examination is a sample of ultrapure water if the electrical signals acquired from the signal generator 2 and from the measuring device based on cyclic voltammetry 3 correspond to one of the second reference measurements of the reference measurement model;
- detect, on the other hand, that a water sample currently under examination is not an ultrapure water sample if the electrical signals acquired from the signal generator 2 and from the measuring device based on cyclic voltammetry 3 do not correspond to one of the second reference measurements of the reference measurement model; and
- determine microbiological characteristics of a water sample currently under examination, in terms of bacterial species and/or endotoxins present in said water sample currently under examination and relative concentrations, on the basis of a statistical distance of the electrical signals acquired from the signal generator 2 and from the measuring device based on cyclic voltammetry 3 with respect to the first and second reference measurements of the reference measurement model.

Preferably, the control and processing means 4 are configured to perform the above-mentioned functions of detection of samples of pure/ultrapure water and microbiological characterization of the water samples under examination by exploiting statistical techniques of multivariate analysis, conveniently statistical techniques of multivariate analysis based on unsupervised models, for example principal component analysis - PCA and/or partial least square (PLS) regression.

Conveniently, the group formed by the signal generator 2 and the measuring device based on cyclic voltammetry 3 expresses the lowest possible output noise, i.e. such that the spectral density of the equivalent generator of noisy current and the spectral density of the equivalent generator of input voltage are lower than

$$10^{-13} A/\sqrt{Hz} \text{ and } 10nV/\sqrt{Hz}$$ respectively. In other words, the signal generator 2 and the measuring device based on cyclic voltammetry 3 are electrically designed so that the spectral density of the corresponding equivalent generator of noisy current and the spectral density of the corresponding equivalent generator of input voltage are lower than $10^{-13} A/\sqrt{Hz}$ and

$$10nV/\sqrt{Hz}$$ respectively. In particular, both the signal generator 2 and the measuring device based on cyclic voltammetry 3 comprise operational amplifiers characterized by a high input impedance and a low output impedance. This guarantees that the signal is transferred as accurately as possible without the introduction of errors, thus resulting in a high signal-noise ratio.

More conveniently, it is the assembly formed of the signal generator 2, the measuring device based on cyclic voltammetry 3 and also the power supply circuit and acquisition circuit (used by the control and processing means 4 to acquire the output voltage $V_{OUT}$ from the measuring device based on cyclic voltammetry 3 and the electrical voltage $V_{IN}$ generated by the signal generator 2) which is designed overall in such a way that the spectral density of the corresponding equivalent generator of noisy current and the spectral density of the corresponding equivalent generator of input voltage are lower than

$$10^{-13} A/\sqrt{Hz} \text{ and } 10nV/\sqrt{Hz}$$ respectively.

In detail, the power supply circuit is preferably a circuit of the split supply type which is configured to receive in input a positive electrical voltage $2V^+$ (for example equal to 9 V) and to split said voltage $2V^+$ into the two above-mentioned power supply (or polarisation) voltages $V^+$ and $V^-$ (having, for example, as previously said, values of +4.5 V and -4.5 V respectively). In further detail, the power supply circuit preferably comprises:

- two input branches by means of which, in use, the positive electrical voltage $2V^+$ is received in input;
- a capacity (for example with value of 10 $\mu$F) connected between the two input branches and configured to decouple said two input branches;

- a voltage divider comprising two resistors (for example with value of 10 kΩ), in which a first resistor is connected between a first input branch and an intermediate node of the voltage divider and the second resistor is connected between the second input branch and said intermediate node of the voltage divider; and
- an operational amplifier having the non-inverting input (+) connected to said intermediate node of the voltage divider and the inverting input (-) connected to the output of said operational amplifier.

In order to obtain a voltage equal to $V^+$ and $V^-$ at the ends of the resistors, there must be a null voltage value between them. This is achieved by means of a buffer. The voltage values thus obtained are also used for powering the same operational amplifier of said power supply circuit. In this way two voltage values are obtained $V^+$ and $V^-$ and the output of the operational amplifier is used as virtual earth of the entire circuit.

As previously explained in detail, the measuring device based on cyclic voltammetry 3, in order to provide a cyclo-voltammogram in output, in use receives an appropriate input signal, conveniently of triangular form, provided by the signal generator 2.

Furthermore, again as explained in detail previously, the measuring device based on cyclic voltammetry 3 is configured to allow the signal of interest to be taken directly from the electrochemical cell 31, in particular from the working electrode 312, by means of a transimpedance configuration (i.e. by means of the current-voltage converter 33 which is, as previously explained, a transimpedance amplifier). A voltage signal is then drawn and, dividing by the impedance of the circuit, current values are obtained.

Moreover, again as explained in detail previously, the control and processing means 4 conveniently comprise a microcontroller for correct acquisition and storage (and, preferably also display) of the output voltage $V_{OUT}$ from the measuring device based on cyclic voltammetry 3 and of the electrical voltage $V_{IN}$ generated by the signal generator 2. The signal in output from the measuring chain conveniently has a waveform that varies between $\pm 300$ mV, while the microcontroller is only able to control positive signals. For this reason an offset is conveniently added both to the output voltage $V_{OUT}$ and to the triangular wave $V_{IN}$, so that they can be correctly acquired by the microcontroller. The offset is applied, preferably, by means of a differential instrumentation amplifier (in particular, the signal to be recorded and displayed is conveniently placed on the negative terminal, while the offset is conveniently placed on the positive terminal of the differential instrumentation amplifier).

Therefore, the signal generator 2, the measuring device 3 and also the above-mentioned power supply and acquisition circuits are electrically designed so that the spectral density of the corresponding equivalent generator of noisy current and the spectral density of the corresponding equivalent generator of input voltage are lower than $10^{-13} A / \sqrt{Hz}$ and $10 nV / \sqrt{Hz}$ respectively. In particular, the signal generator 2, the measuring device based on cyclic voltammetry 3 and also the above-mentioned power supply and acquisition circuits comprise electronic components and operational amplifiers electrically designed so that the spectral density of the equivalent generator of noisy current and the spectral density of the equivalent generator of input voltage are lower than $10^{-13} A / \sqrt{Hz}$ and $10 nV / \sqrt{Hz}$ respectively.

Figure 4 shows a specific preferred embodiment of the system 1 shown in Figure 2 and previously described in detail.

In particular, Figure 4 shows:

- a portable electronic device 7 which includes the signal generator 2, the control and processing means 4 and the user interface 5; and
- a portable measuring device 8 which includes the measuring device based on cyclic voltammetry 3.

As shown in Figure 4, the electronic device 7 is connected to the measuring device 8 by means of a cable 9 so that, in use, the signal generator 2 supplies to the measuring device based on cyclic voltammetry 3 the electrical voltage $V_{IN}$ with triangular waveform and the control and processing means 4 acquire from said measuring device based on cyclic voltammetry 3 the output voltage $V_{OUT}$.

In detail, in the measuring device 8 the container 311 of the electrochemical cell 31 of the measuring device based on cyclic voltammetry 3 is produced in the shape of a test tube 81, for example made of glass or plastic, with substantially cylindrical shape closed at the bottom and open at the top, hence said test tube 81 is substantially cup-shaped. Conveniently, the test tube 81 could also be produced in other different shapes, for example it could be in the shape of a finger or a tube. The bottom of the test tube 81 shown in Figure 4 is flat, but it could also conveniently be rounded. The upper opening of the test tube 81 is hermetically closed by a plug 82, for example made of plastic, which houses within it the operational amplifier 32 and the current-voltage converter 33.

Furthermore, in the measuring device 8 the working electrode 312, the counter electrode 313 and the reference electrode 314 are made in the form of SPEs 83 which extend from a lower portion of the plug 82 to the inside of the test tube 81.

Lastly, the plug 82 carries on an upper portion:

- a first connector 84, for example a port, designed to be connected to the cable 9; and
- a second connector 85 designed to be connected, by means of luer lock connection, to an aseptic sampling port for introducing a water sample to be analysed into the test tube 81 in a completely aspetic manner.

In the light of the above, the system 1 produced by means of the electronic device 7 and the measuring device 8 is a portable analysis system which, in use, requires an operator to:

- withdraw in a completely aseptic manner a water sample to be analysed connecting, by means of luer lock connection, the measuring device 8 (in particular, the second connector 85 of said measuring device 8) to an aseptic sampling port;
- connect, by means of the cable 9, the measuring device 8 to the electronic device 7; and
- operate the electronic device 7 in order to analyse the water sample present inside the measuring device 8 (in particular, inside the test tube 81).

The system 1 produced by means of the electronic device 7 and the measuring device 8 is therefore completely aspetic (i.e. it guarantees the aseptic conditions necessary for the analysis) and prevents any contamination of the water samples to be analysed.

Preferably, the measuring device 8 is a disposable device. In particular, the test tube 81 and the SPEs 83 are conveniently disposable devices.

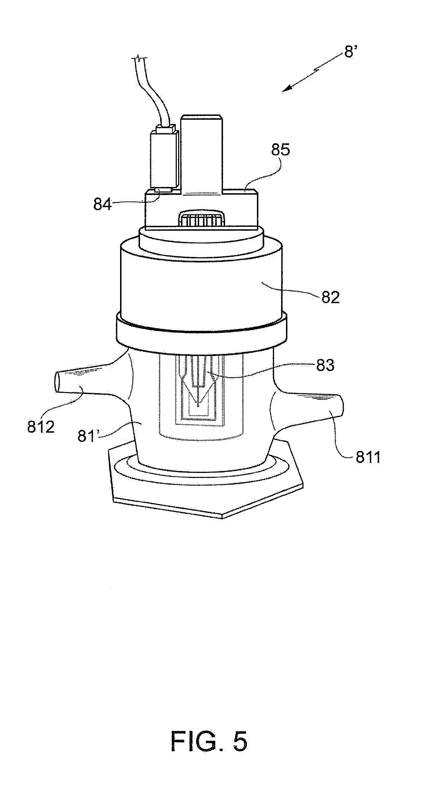

Figure 5 shows an alternative embodiment of the measuring device 8. In particular, Figure 5 shows a measuring device 8' which differs from the measuring device 8 shown in Figure 4 and previously described due to the fact that it is configured to be connected directly to a production and/or treatment and/or distribution plant for pure and/or ultrapure water, for example a plant designed to supply pure and/or ultrapure water intended for haemodialysis.

In detail, the measuring device 8' comprises a test tube 81' which has all the characteristics of the test tube 81 of the measuring device 8, with the exception of the fact that said test tube 81' also includes:

- an inlet 811, which is arranged on the lateral wall of the test tube 81' and is designed to

  - be connected to a production and/or treatment and/or distribution plant for pure and/or ultrapure water to receive water to be analysed, and
  - let into the test tube 81' the water received from the production and/or treatment and/or distribution plant for pure and/or ultrapure water; and

- an outlet 812, which is arranged on the lateral wall of the test tube 81' in a higher position than the inlet 811 and is designed to

  - be connected to the production and/or treatment and/or distribution plant for pure and/or ultrapure water to supply the analysed water to the latter, and

- let out the analysed water present inside the test tube 81'.

Conveniently, with the measuring device 8' a non-portable electronic device 7 could also be used, for example a fixed electronic device remotely connected to the measuring device 8'.

In the light of the above, the system 1 provided by means of the electronic device 7 and the measuring device 8' is an on-line analysis system which, in use, is connected directly to a production and/or treatment and/or distribution plant for pure and/or ultrapure water and analyses, in on-line mode and in real time, the water produced and/or treated and/or distributed by said plant. In this way the system 1 provided by means of the electronic device 7 and the measuring device 8' analyses, in a totally reliable, independent and autonomous manner, water samples taken directly from the production and/or treatment and/or distribution plant for pure and/or ultrapure water, generating alarms if one or more water samples analysed do not meet predetermined quality conditions for the pure and/or ultrapure water. Said solution allows prompt correction interventions to be carried out to correct the quality of the pure and/or ultrapure water.

Below the present invention will be compared with the teachings of the articles "Rapid electrochemical detection of polyaniline-labeled Escherichia coli O157:H7" by Setterington E. B. et al. (which will be referred to below, for the sake of brevity, as Setterington) and "Quantitation of bacteria through absorption of intracellular biomolecules on carbon paste and screen-printed carbon electrodes and voltammetry of redox-active probes" by Agnes Obuchowska (which will be referred to below, for the sake of brevity, as Obuchowska), US 2006/0228738 A1 and, lastly, "Bioelectronics for Amperometric Biosensors" by Jaime Punter Villagrasa et al. (which will be referred to below, for the sake of brevity, as Villagrasa).

Setterington presents a study based on the identification of one single bacterium (i.e. Escherichia coli O157:H7) via a chemical method different from the one used by the present invention which, on the other hand, detects one or more bacterial species and/or endotoxins present in a sample analysed. Setterington teaches the use of functionalized electrodes, i.e. electrodes that necessarily require the use of further cells/substances/ materials that allow said electrodes to function. Furthermore Setterington also teaches labelling of the Escherichia coli O157:H7 bacteria, whereas the present invention does not provide for any treatment of the water sample under examination prior to the measurements based on cyclic voltammetry.

Contrary to Setterington, the present invention does not provide for any functionalization of the electrodes and this makes the measurement method according to the present invention more stable and more reproducible. The lack of functionalization of the electrodes further entails greater ease of cleaning of the latter because materials are not deposited on them. In simple terms, the system according to the present invention allows the desired result to be obtained (i.e. the characterization of pure and ultrapure water) in a much simpler manner than Setterington. In fact, according to the present invention no further variables are added (for example the chemical quality of the material deposited on the electrodes) which could introduce errors into the measuring chain.

Furthermore, the system according to Setterington has a specific application in the analysis of food or water intended for human consumption, whereas the application of the present invention is for pure and ultrapure water which, as is widely known, has completely different chemical-physical characteristics; in particular, as explained previously, pure and ultrapure water has chemical and microbiological characteristics well defined in the various reference standards previously indicated.

The study performed by Obuchowska is aimed at determining the quantity of bacteria in a sample. A fundamental difference compared to the present invention is that the sample examined by Obuchowska is not pure or ultrapure water (as in the case of the present invention), but drinking water. Furthermore in Obuchowska the sample is treated before carrying out the measurements, since without said treatment the measurements would not produce results. The present invention, on the other hand, does not comprise any treatment of the sample apart from the application of an electrical potential, from which the potential generated by the sample is measured. In particular, the present invention does not entail any modification of the sample characteristics before carrying out the measurements based on cyclic voltammetry.

As regards US 2006/0228738 A1, said document provides for the use of a gas inside the measuring chamber, the latter characteristic being absent in the present invention. Moreover, the use of a gas entails a series of consequences in terms of reproducibility of the measurement, since the conditions can vary and are subject to variations according to the characteristics of the gas, the method of introduction into the chamber, etc..

The advantage of having a simpler system that does not comprise additional elements either on the electrodes or within the electrochemical cell is that a more accurate measurement method is obtained, because there is a lower possibility of error, consequently it is more reproducible.

Contrary to the present invention, also in the case of US 2006/0228738 A1 the electrodes are functionalized.

As regards Villagrasa, it should be noted that this document is limited to providing only an overview of the state of the art in the sector of biosensors and bioelectronics, but without actually anticipating the characteristics of the system according to the present invention.

Lastly, it is important to underline the fact that none of the above-mentioned prior documents describe the use of a working electrode made of gold, a counter electrode made of platinum and a reference electrode made of Silver chloride, or a device that expresses the lowest possible noise in output, i.e. such that the spectral density of the equivalent generator of noisy current and the spectral density of the equivalent generator of voltage in input are lower than $10^{-13} A/\sqrt{Hz}$ and $10nV/\sqrt{Hz}$ respectively.

From the preceding description, the numerous technical advantages of the present invention are immediately evident.

Firstly, it is important to underline the fact that the system according to the present invention is able to characterize water samples extremely rapidly; in the case of the on-line analysis system provided by means of the electronic device 7 and the measuring device 8', even in real time.

Furthermore, the system according to the present invention is a low cost system which is able to perform the microbiological characterization of pure and ultrapure water in an extremely efficient, reliable and reproducible manner.

Lastly, the system according to the present invention is able to perform the microbiological characterization of pure and ultrapure water always guaranteeing aseptic conditions for analysis/examination of the water.

As previously said, the present invention can be advantageously exploited for the microbiological characterization of pure and ultrapure water intended, for example, for haemodialysis.

In conclusion, it is clear that various modifications can be made to the present invention, all falling within the protective scope of the invention as defined in the attached claims.

**Claims**

1. A system for pure and ultrapure water characterisation (1), comprising:

   • a signal generator (2) configured to generate an electrical voltage having a predetermined waveform such that to allow to perform measurements based on cyclic voltammetry;
   • a measuring device (3;8;8') configured to perform measurements based on cyclic voltammetry on a water sample under examination (6) on the basis of the electrical voltage generated by the signal generator (2); and
   • processing means (4) configured to characterize the water sample under examination (6) on the basis of the measurements based on cyclic voltammetry performed by the measuring device (3;8;8') on said water sample under examination (6);

wherein the measuring device (3;8;8') comprises:

• an electrochemical cell (31), which includes

- a container (311) designed to contain the water sample under examination (6), and
- a working electrode (312), a counter electrode (313) and a reference electrode (314) arranged such as to be immersed, at least partially, in the water sample under examination (6) contained in the container (311);

• a first operational amplifier (32), which includes

- a non-inverting input designed to receive the electrical voltage generated by the signal generator (2),
- an inverting input connected to the reference electrode (314),
- an output connected to the counter electrode (313), and
- a feedback capacity (321) arranged between the inverting input and the output, thereby resulting in said first operational amplifier (32) being configured to apply to the electrochemical cell (31) the electrical voltage generated by the signal generator (2) to perform the measurements based on cyclic voltammetry on the water sample under examination (6); and

• a current-voltage converter (33), which is

- connected to the working electrode (312) to receive an electrical current induced on said working electrode (312) by application to the electrochemical cell (31) of the electrical voltage generated by the signal generator (2), and
- configured to convert said electrical current into a corresponding electrical voltage, thereby providing an electrical output voltage;

and wherein the processing means (4) are configured to detect whether the water sample under examination (6) is a pure or ultrapure water sample on the basis of:

• the electrical voltage generated by the signal generator (2);
• the electrical output voltage supplied by the current-voltage converter (33); and
• a reference measurement model generated on the basis of measurements based on cyclic voltammetry performed by the measuring device (3;8;8') on

- first reference water samples having first chemical and microbiological characteristics which, according to a given reference standard, characterize pure water, and
- second reference water samples having second chemical and microbiological characteristics which, according to said given reference standard, characterize ultrapure water.

2. The system of claim 1, wherein said first and second reference water samples contain known concentrations of bacteria and/or endotoxins; and wherein the processing means (4) are configured to:

• detect one or more bacterial species and/or one or more endotoxins present in the water sample under examination (6); and
• determine, for each bacterial species and/or endotoxin detected, a respective concentration.

3. The system according to claim 1 or 2, wherein the processing means (4) are configured to generate the reference measurement model during a calibration phase of the system (1).

4. The system according to any claim 1-3, wherein the processing means (4) are configured to characterize the water sample under examination (6) using one or more statistical techniques of multivariate analysis.

5. The system according to any preceding claim, wherein the current-voltage converter (33) is a transimpedance amplifier which includes:

• a second operational amplifier (331) which comprises

- a non-inverting input connected to ground,
- an inverting input connected to the working electrode (312), and
- an output on which the electrical output voltage is supplied; and

• a feedback resistor (332) arranged between the inverting input and the output of the second operational amplifier (331).

6. The system according to any preceding claim, wherein the electrodes (312,313,314) of the electrochemical cell (31) are screen-printed electrodes (83).

7. The system according to any preceding claim, wherein the working electrode (312) is made of gold, the counter electrode (313) is made of platinum, and the reference electrode (314) is made of silver chloride.

8. The system according to any preceding claim, wherein the signal generator (2) and the measuring device (3;8;8') are electrically configured so that a spectral density of a corresponding equivalent generator of noisy current and a spectral density of a corresponding equivalent generator of input voltage are lower than $10^{-13} A / \sqrt{Hz}$ and $10nV / \sqrt{Hz}$ respectively.

9. The system according to any preceding claim, wherein the processing means (4) are configured to generate information indicative of characteristics of said water sample under examination (6);
and wherein the processing means (4) and the signal generator (2) are integrated in one and the same electronic device (7), which is connectable to the measuring device (8;8') and which includes also a user interface (5) configured to:

• display the information generated by the processing means (4); and
• allow a user to set the predetermined waveform of the electrical voltage generated by the signal generator (2).

10. The system of claim 9, wherein the electronic device (7) and the measuring device (8;8') are portable devices; and wherein the measuring device (8;8') comprises a connector (85) designed to be connected, by means of luer lock connection, to an aseptic port for introduction inside the measuring device (8;8') of a water sample to be characterized.

11. The system according to any claim 1-9, wherein the measuring device (8') is configured to be connected to a plant for the production and/or treatment and/or distribution of pure and/or ultrapure water.

TABLE - MICROBIOLOGICAL CONTROLS FOR HAEMODIALYSIS

| TEST | MAINS WATER | | TREATED WATER (beginning and end of distribution circuit) | | STANDARD DIALYSATE | | ULTRAPURE DIALYSATE | |
|---|---|---|---|---|---|---|---|---|
| | Reference values | Frequency | Reference values | Frequency | Reference values | Frequency | Reference values | Frequency |
| Bacteria UFC/mL at 22° C | <100 | every 6 months | <100 | once a month | <100 | every 4 months in every monitor | <0.1 | every 4 months in every monitor on which treatments are performed |
| Moulds and yeasts/mL | - | - | <10 | every 6 months | <10 | | 0 | on-line |
| Endotoxins UI/mL | - | - | <0.25 | once a month | <0.25 | | <0.03 | |

FIG. 1

EP 3 001 193 A1

| Signal generator | → | Measuring device based on cyclic voltammetry |

FIG. 2

FIG. 4

FIG. 3

EP 3 001 193 A1

FIG. 5

Europäisches
Patentamt

European
Patent Office

Office européen
des brevets

**EUROPEAN SEARCH REPORT**

Application Number

EP 15 18 6999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | SETTERINGTON E B ET AL: "Rapid electrochemical detection of polyaniline-labeled Escherichia coli O157:H7", BIOSENSORS AND BIOELECTRONICS, ELSEVIER BV, NL, vol. 26, no. 5, 15 January 2011 (2011-01-15), pages 2208-2214, XP027580141, ISSN: 0956-5663 [retrieved on 2010-09-25] * the whole document * | 1-11 | INV. G01N33/18 ADD. G01N27/48 |
| X,D | AGNES OBUCHOWSKA: "Quantitation of bacteria through adsorption of intracellular biomolecules on carbon paste and screen-printed carbon electrodes and voltammetry of redox-active probes", ANALYTICAL AND BIOANALYTICAL CHEMISTRY, SPRINGER, BERLIN, DE, vol. 390, no. 5, 20 January 2008 (2008-01-20), pages 1361-1371, XP019584887, ISSN: 1618-2650 * the whole document * | 1-11 | |
| X,D | US 2006/228738 A1 (ALOCILJA EVANGELYN [US] ET AL) 12 October 2006 (2006-10-12) * figure 1; example 1 * | 1-11 | |

-----

-----

-/--

TECHNICAL FIELDS
SEARCHED      (IPC)

G01N

The present search report has been drawn up for all claims

| Place of search | Date of completion of the search | Examiner |
|---|---|---|
| The Hague | 15 January 2016 | Zwerger, Markus |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

## EUROPEAN SEARCH REPORT

Application Number

EP 15 18 6999

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | Jaime Punter ET AL: "Bioelectronics for Amperometric Biosensors" In: "State of the Art in Biosensors - General Aspects", 13 March 2013 (2013-03-13), InTech, XP055166557, ISBN: 978-9-53-511004-0 DOI: 10.5772/52248, * figure 6 * | 1-11 | |
| A | US 2009/277805 A1 (AMEMIYA SHIGERU [US] ET AL) 12 November 2009 (2009-11-12) * paragraphs [0068], [0095] - [0105] * | 1-11 | |

TECHNICAL FIELDS
SEARCHED (IPC)

The present search report has been drawn up for all claims

| Place of search | Date of completion of the search | Examiner |
|---|---|---|
| The Hague | 15 January 2016 | Zwerger, Markus |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 15 18 6999

This annex lists the patent family members relating to the patent documents cited in the above-mentioned European search report.
The members are as contained in the European Patent Office EDP file on
The European Patent Office is in no way liable for these particulars which are merely given for the purpose of information.

15-01-2016

| Patent document cited in search report | Publication date | Patent family member(s) | Publication date |
|---|---|---|---|
| US 2006228738 A1 | 12-10-2006 | NONE | |
| US 2009277805 A1 | 12-11-2009 | NONE | |

EPO FORM P0459

For more details about this annex : see Official Journal of the European Patent Office, No. 12/82

## REFERENCES CITED IN THE DESCRIPTION

**Patent documents cited in the description**

- US 20060228738 A1

**Non-patent literature cited in the description**

- *Italy the Legislative Decree no. 31,* 02 February 2001
- Rapid electrochemical detection of polyaniline-labeled Escherichia coli O157:H7. **SETTERINGTON E. B. et al.** Biosensors and Bioelectronics. Elsevier BV, 15 January 2011, vol. 26, 2208-2214
- Quantitation of bacteria through absorption of intracellular biomolecules on carbon paste and screen-printed carbon electrodes and voltammetry of redox-active probes. **AGNES OBUCHOWSKA.** Analytical and Bioanalytical Chemistry. Springer, 20 January 2008, vol. 390, 1361-1371
- Bioelectronics for Amperometric Biosensors. **JAIME PUNTER VILLAGRASA et al.** State of the Art in Biosensors - General Aspects. 13 March 2013
- **SETTERINGTON E. B. et al.** which will be referred to below, for the sake of brevity, as Setterington. *Rapid electrochemical detection of polyaniline-labeled Escherichia coli O157:H7*
- **AGNES OBUCHOWSKA.** which will be referred to below, for the sake of brevity, as Obuchowska. *Quantitation of bacteria through absorption of intracellular biomolecules on carbon paste and screen-printed carbon electrodes and voltammetry of redox-active probes*
- **JAIME PUNTER VILLAGRASA et al.** which will be referred to below, for the sake of brevity, as Villagrasa. *Bioelectronics for Amperometric Biosensors*